(19) Europäisches Patentamt / European Patent Office / Office européen des brevets

(11) **EP 4 157 291 B1**

(12) **EUROPEAN PATENT SPECIFICATION**

(45) Date of publication and mention of the grant of the patent:
**13.08.2025 Bulletin 2025/33**

(21) Application number: **21726407.6**

(22) Date of filing: **19.05.2021**

(51) International Patent Classification (IPC):
*A61K 31/728* $^{(2006.01)}$    *A61K 9/00* $^{(2006.01)}$
*A61K 47/00* $^{(2006.01)}$    *A61P 27/02* $^{(2006.01)}$

(52) Cooperative Patent Classification (CPC):
**A61K 31/728; A61K 9/0048; A61P 27/02**

(86) International application number:
**PCT/EP2021/063314**

(87) International publication number:
**WO 2021/239544 (02.12.2021 Gazette 2021/48)**

(54) **OPHTHALMIC COMPOSITION BASED ON HYALURONIC ACID TO PROTECT FROM DAMAGES CAUSED BY ENVIRONMENTAL POLLUTION**

OPHTHALMISCHE ZUSAMMENSETZUNG AUF HYALURONSÄUREBASIS ZUM SCHUTZ VOR SCHÄDEN DURCH UMWELTVERSCHMUTZUNG

COMPOSITION OPHTALMIQUE À BASE D'ACIDE HYALURONIQUE POUR PROTÉGER DES DOMMAGES PROVOQUÉS PAR LA POLLUTION ENVIRONNEMENTALE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **27.05.2020 IT 202000012535**

(43) Date of publication of application:
**05.04.2023 Bulletin 2023/14**

(73) Proprietor: **Colombo, Stefano**
**20144 Milano (IT)**

(72) Inventors:
• **PORCHETTI, Anna**
**20021 Bollate MI (IT)**
• **COLOMBO, Stefano**
**20144 Milano (IT)**

(74) Representative: **De Santis, Giovanni et al**
**c/o Giambrocono & C. S.p.A.**
**Via Rosolino Pilo, 19/B**
**20129 Milano (IT)**

(56) References cited:
EP-A1- 3 756 651    WO-A1-2016/162809
WO-A1-2016/181342    IT-A1- UB20 155 351

• **MONTEFARMACO: "Iridina Lubricating Drops", 12 December 2019 (2019-12-12), XP055769721, Retrieved from the Internet <URL:https://montefarmaco.com/en/ophthalmic/iridina/iridina-lubricating-drops/> [retrieved on 20210128]**
• **MONTFARMACO: "iridina gocce lubrificanti", 2019, XP055769645, Retrieved from the Internet <URL:https://www.montefarmaco.com/wp-content/uploads/2017/02/Foglietto-Illustrativo-Iridina-Gocce-Lubrificanti.pdf> [retrieved on 20210128]**
• **JI-MIN LI ET AL: "Hyaluronic acid-dependent protection against UVB-damaged human corneal cells : Hyaluronic Acid-Dependent Protection on UVB Damage", ENVIRONMENTAL AND MOLECULAR MUTAGENESIS., vol. 54, no. 6, 27 June 2013 (2013-06-27), GB, pages 429 - 449, XP055602162, ISSN: 0893-6692, DOI: 10.1002/em.21794**
• **TAKASHI KOJIMA ET AL: "The Effects of High Molecular Weight Hyaluronic Acid Eye Drop Application in Environmental Dry Eye Stress Model Mice", INTERNATIONAL JOURNAL OF MOLECULAR SCIENCES, vol. 21, no. 10, 15 May 2020 (2020-05-15), pages 3516, XP055769490, DOI: 10.3390/ijms21103516**

• IN FOCUS: "Iridina  Lubricating Drops in relieving eye discomfort and irritation", 1 July 2020 (2020-07-01), XP055769498, Retrieved from the Internet <URL:https://springerhealthcare.it/wp-content/uploads/2020/07/MNT151-20-In-Focus-Anno-XXIII-n.-13-july-2020.pdf> [retrieved on 20210127]

**Description**

The present invention relates to an ophthalmic composition in the form of ocular drops consisting of hyaluronic acid or a physiologically acceptable salt thereof, preferably sodium salt, in admixture with excipients for ophthalmic use, for use to protect the eyes from damages caused by excessive exposure to blue light.

The eye is a delicate organ. Its wet surface is widely exposed to the surrounding environment and therefore it is more susceptible to environmental pollution than other parts of the body. The response of the eyes to environmental pollution may range from mild irritation up to severe irritation which may lead to chronic pain. Moreover, the use of contact lenses makes the eyes more sensitive to such effects. Air pollution (outdoor pollution) is mainly due to the exhaust fumes from cars and other veihicles. Air pollutants which are commonly found in metropolitan cities include carbon monoxide, nitrogen dioxide, sulfur dioxide, arsenic, asbestos, benzene, lead, chlorofluorohydrocarbons, particulates and dioxin. The exposure of the eyes to such pollutants for more than 4 hours may cause strong irritations.

The subjects who are more exposed to these ocular irritations are those working outdoor (e.g. traffic policemen, construction workers, toll collectors, etc.).

Light pollution (indoor pollution) is another form of environmental pollution.

Sunlight (white light) is divided in several components - red, orange, yellow, green, blue, indigo and violet light - all characterized by different energies and wavelengths. Red light rays have longer wavelengths and less energy, while blue light rays have shorter wavelengths and more energy.

The largest source of blue light is sunlight. Additional sources are fluorescent light, compact fluorescent light (CFL) bulbs, LED lights, flat screen LED televisions, computer monitors, smartphones and tablet screens.

Almost all the blue light the cornea is exposed to reaches the retina. This light affects vision and may prematurely age the eyes. Blue light from computer screens and digital devices may cause eyestrain, ocular dryness, irritation and difficulty focusing. Spending many hours in front of computers, smartphones and tablet monitors may cause eye problems.

People who are more exposed to damages caused by light pollution are those staying in a closed environment with light pollution from artificial light and/or using electronic devices and display screens emitting blue light for many consecutive hours.

Ocular damages are different depending on the wavelength of the light.

UV light as well as IR light are mainly adsorbed by the cornea and the anterior chamber of the eye. A high exposure to UV or IR light causes damages to the corneal cells.

Conversely, blue light penetrates more deeply into the eye and causes irreversible oxygen-dependent damages to the retina.

Thus there is the need to protect the subjects in need thereof from the damages to the eyes caused by environmental pollution, i.e. atmospheric as well as light pollution.

Iridina® Lubricating Drops is a product available on the market since many years. It is a sterile solution for ophthalmic use at physiological pH containing sodium hyaluronate, boric acid and sodium tetraborate (buffer) and sodium chloride. Sodium hyaluronate confers to the drops mucomimetic and viscoelastic properties thereby assuring an adequate lubrication of the ocular surface. In addition, sodium hyaluronate is able to retain water and maintain wet the ocular surface so increasing the stability of the lacrimal film.

Due to its composition based on hyaluronic acid, Iridina® Lubricating Drops is used as hydrating and lubricant for the ocular surface and gives temporary relief from burning, irritation and discomfort related to ocular dryness of any origin.

The Applicant has now found that hyaluronic acid and salts thereof have also a protective effect from the damages caused by a high level of exposure to blue light. Hyaluronic acid and salts thereof, mainly the sodium salt, are widely used in formulations for ophthalmic use.

Among its known uses, also the protective effect against the UV light may be cited.

Pauloin et al. (Molecular Vision, 2009, 15:577-583) disclose that high molecular weight hyaluronic acid (1500 kDa) is able to protect corneal epithelial cells from the cytotoxic effects induced by ultraviolet B (UVB) light. This protective effect has been subsequently confirmed by Li et al. (Environmental and Molecular Mutagenesis, 2013, 54:429-449) for high molecular weight hyaluronic acid (1000 kDa) compared to low molecular weight hyaluronic acid (100 kDa), which resulted to be ineffective.

WO 2016/181342 discloses an ophthalmic composition comprising sodium hyaluronate useful to protect the surface of the eye from adverse environmental conditions such as pollution and excessive exposure to sunlight. IT102015000065559 discloses an ophthalmic composition containing hyaluronic acid for re-epithelizing and lubricating cornea and conjunctiva with protective action. Kojima et al. (International Journal of Molecular Sciences, 2020, 21:3516) discloses the beneficial effects of high molecular weight hyaluronic acid eye drop application in environmental dry eye stress model mice. WO 2016/162809 discloses the ophthalmic use of glycosaminoglycan esters.

According to the Applicant's best knowledge, the use of hyaluronic acid or salts thereof in ocular drops to protect from the damages caused by the excessive exposure to blue light is not known.

Consistently, an ophthalmic solution sold under the tradename Lumixa®, based on sodium hyluronate and crocine, indicated for ocular dryness and to limit the ocular damages caused by environmental pollution, attributes its efficacy against the damages from environmental pollution only to the presence of crocine.

Therefore, object of the present invention is an ophthalmic composition in the form of ocular drops consisting of hyaluronic acid or a physiologically acceptable salt thereof, preferably sodium salt, in admixture with excipients for ophthalmic use for use to protect the eyes from damages caused by excessive exposure to blue light. Hyaluronic acid is preferably high molecular weight hyaluronic acid, particularly having a molecular weight from 700 to 1500 kDa.

Hyaluronic acid with molecular weight 900-1000 kDa is particularly preferred.

The use of a hyaluronic acid salt, particularly sodium hyaluronate, is still more preferred.

The amount (expressed as concentration) of hyaluronic acid or a pharmaceutically acceptable salt thereof is generally from 0.1 to 0.5% w/v, more preferably from 0.2 to 0.4% w/v, still more preferably is 0.4% w/v.

Typically the ophthalmic composition contains one or more excipients suitable for the preparation of ophthalmic products.

Solvents such as water, isotonic agents such as sodium chloride, buffering agents such as boric acid/sodium borate, to keep the pH at a physiological value (pH about 7.2) and optionally preservatives and/or chelating agents are particularly suitable.

All the excipients which may be used are normally used in ophthalmic formulations and are well known to the skilled in the art.

The choice of the excipients depends on the selected type of ophthalmic composition and is within the common knowledge of the skilled in the art.

The composition in the form of drops commercialized by the present Applicant under the tradename Iridina® Lubricating Drops is particularly preferred for the use object of the present invention.

Iridina® Lubricating Drops is a sterile aqueous solution at physiological pH containing 0.4% sodium hyaluronate, boric acid and sodium tetraborate, and sodium chloride.

The efficacy of the protective action of Iridina® Lubricating Drops against blue light was assessed *in vitro* on reconstructed ocular epithelium as ability to prevent the formation of free radicals (ROS) as described in details in the experimental section below.

Experimental section

*In vitro assessment of the protective action of Iridina® Lubricating Drops against blue light on reconstructed ocular epithelium*

The purpose of the test is to assess the *in vitro* protective activity of the tested product following irradiation with blue light. For this reason its efficacy in preventing the formation of radical oxygen species (ROS) on reconstructed ocular epithelium has been tested.

The following materials and methods were used.

*Tissues*

The reconstructed tridimensional tissue of corneal epithelium (RhCE) is a non-keratinized epithelium obtained from normal human keratinocytes. It represents a corneal epithelium model with progressively stratified, but not keratinized, cells.

*Irradiation conditions*

A Wood lamp with irradiance of 4.7 mW/cm$^2$ was used to irradiate the cells with blue light. In order to stimulate the production of ROS the tissues were irradiated with 5, 15, 25 and 30 J/cm$^2$ of blue light.

*Preparation of the sample and DCFDA*

The negative control is represented by the untreated tissues. The positive control is a substance with known antioxidant action. The sample was tested as such. 2',7'-dichlorofluorescein diacetate (DCFDA) was dissolved in dimethylsulfoxide (DMSO) at the concentration of 50 mM and then diluted in the appropriate buffer up to the final concentration of 250 $\mu$M.

*ROS assay*

RhCE tissues were allowed to equilibrate at room temperature for about 15 minutes and then transferred into growth medium and incubated (37°C, 5% $CO_2$) overnight. After the incubation the tissues were moistened and then treated with DCFDA for 30 minutes. DCFDA was then removed, the tissues were washed in PBS, treated with the test sample or with the controls and irradiated with blue light. A fluorimeter reading was finally performed at the excitation wavelength of 485 nm and the emission wavelength of 530 nm.

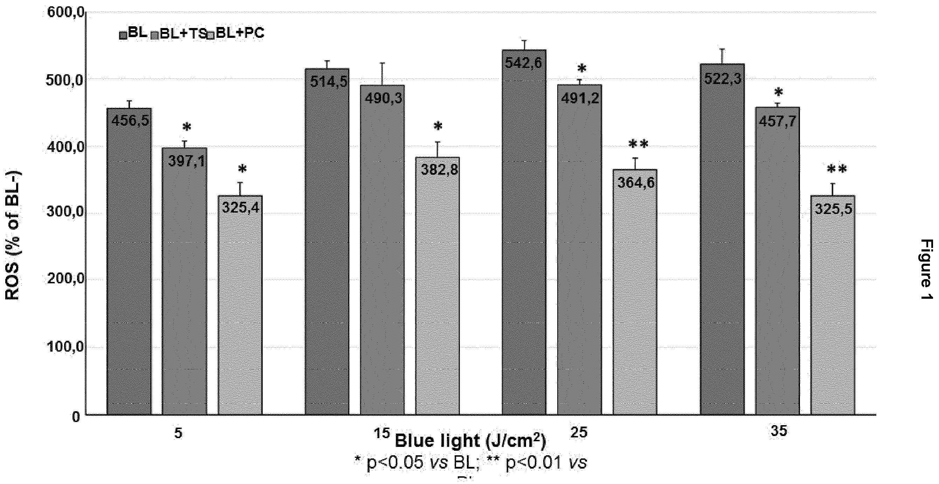

Fig. 1 depicts the assessment of ROS production after irradiation with blue light.

BL = tissues irradiated with the different doses of blue light
TS = tissues treated with the tested product
PC = tissues treated with the positive control

The fluorescence is directly proportional to the amount of ROS produced by the tissues. The percentages were calculated on the basis of the measured fluorescence values and they represent the percentage of ROS compared to the negative control (untreated tissues). The values are expressed as means ± standard deviation. Statistical data processing was performed by Student's t-test. Values of $p < 0.05$ were considered to be significant.

In the tissues treated with the tested product, following irradiation with blue light, a significant reduction in the amount of ROS compared to the untreated tissues was observed. Therefore, the tested product shows *in vitro* protective activity against blue light.

*Clinical observational monitoring to assess the improvement of ocular discomfort*

Iridina® Lubricating Drops was investigated through a clinical observational monitoring on two groups of 50 subjects each, that were subject to sources of atmospheric pollution (outdoor pollution) or light pollution (in indoor environments), to evaluate the improvement of ocular discomfort.

The two groups of 50 subjects each, both males and females and from 18 to 50 years old are:

- 50 subjects who work in the outdoor environment (traffic policemen, construction workers, toll collectors, etc.)
- 50 subjects who stay in indoor environments and are subject to light pollution from artificial light and use electronic devices and display screens emitting blue light for many consecutive hours.

The assessment of the ocular discomfort was quantified by the answers of the subjects to an OSDI (Ocular Surface Disease Index) questionnaire. The questionnaire allows to assess the eye dryness condition on a scale from 0 to 100 and classify it as normal, moderate or severe.

The questionnaire consists of 12 questions to each of which the subjects of the study are asked to reply by indicating a score from 0 to 4.

*Questions*

Have you experienced any of the following during the last week:

1. Eyes sensitive to light
2. Eyes that feel gritty
3. Painful or sore eyes
4. Blurred vision
5. Poor vision

Have your eyes limited you in performing any of the following during the last week:

6. Reading
7. Driving at night
8. Working with a computer
9. Watching TV

Have your eyes felt uncomfortable in any of the following situations during the last week:

10. Windy conditions
11. Staying in very dry places
12. Staying in air conditioned areas

*Scoring system*

For each question the answer can be: None of the time, some of the time, half of the time, most of the time, all of the time.

For each answer the following score was assigned:

0 = non of the time
1 = some of the time
2 = half of the time
3 = most of the time
4 = all of the time

The total score is calculated based on the following formula:

$$OSDI = \frac{(\text{sum of the severity of all given answers}) \times (100)}{(\text{number of given answers}) \times (4)}$$

*OSDI value*

0-12 = normal ocular surface
13-22 = mild dry eye condition
23-32 = moderate dry eye condition
33-100 = severe dry eye condition

*Clinical monitoring study*

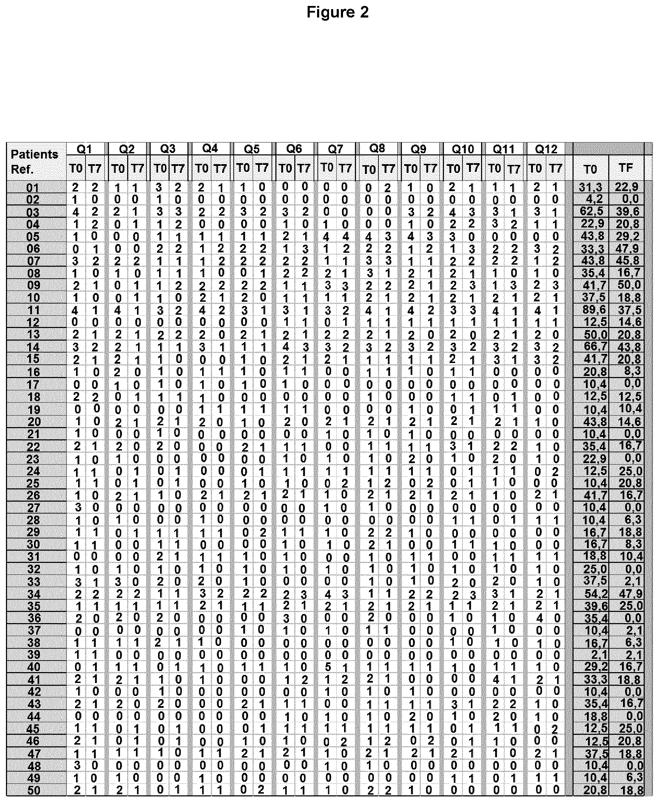

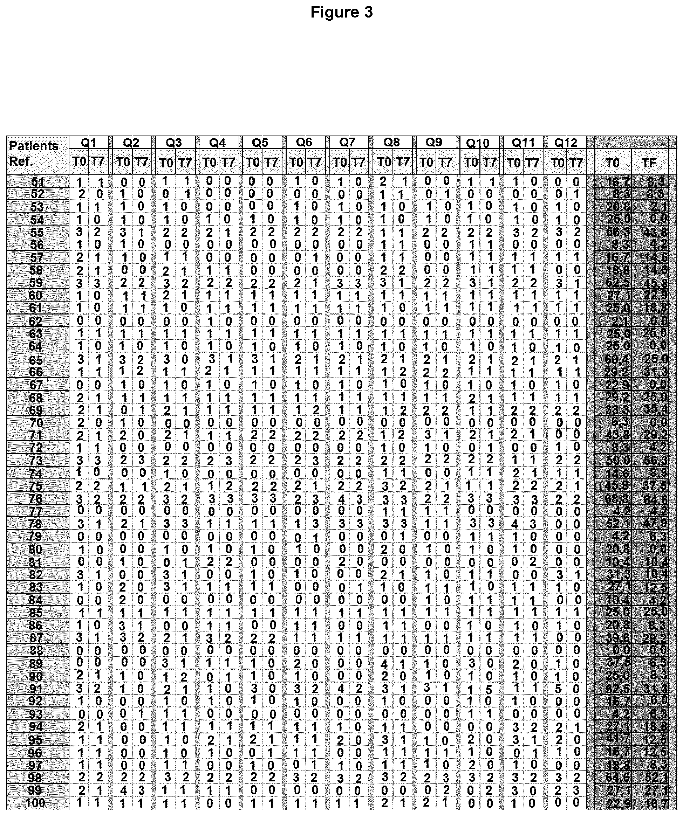

Subjects instilled 1 or 2 drops of Iridina® Lubricating Drops in each eye, at least 2-3 times a day, for 7 days.
The OSDI questionnaires were filled in by each subject of the study at time T0, before starting the treatment, and after 7 days of treatment (T7).
The answers to the OSDI questionnaire for each subject are reported in Fig. 2 (subjects 1-50 - outdoor group) and Fig. 3 (subjects 51-100 - indoor group).

*Statistical analysis of the results and statistical methodology*

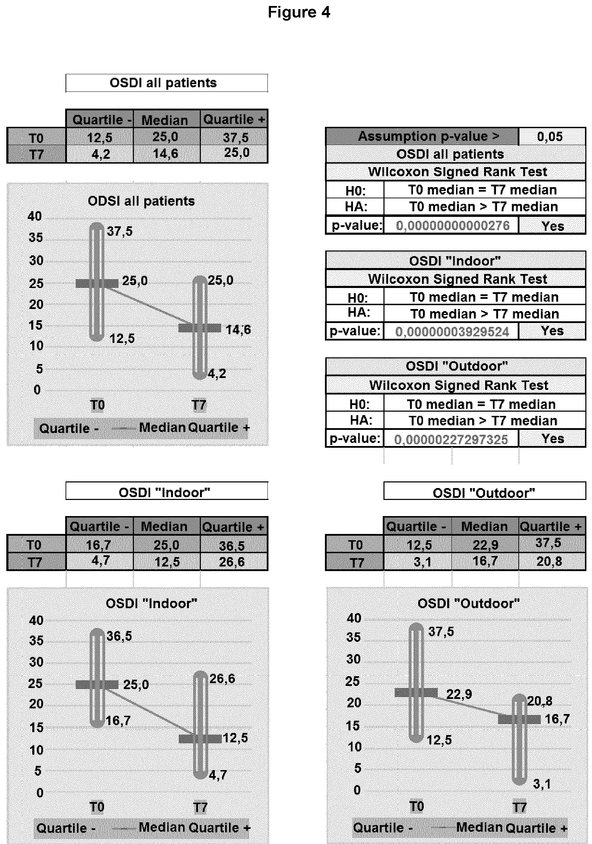

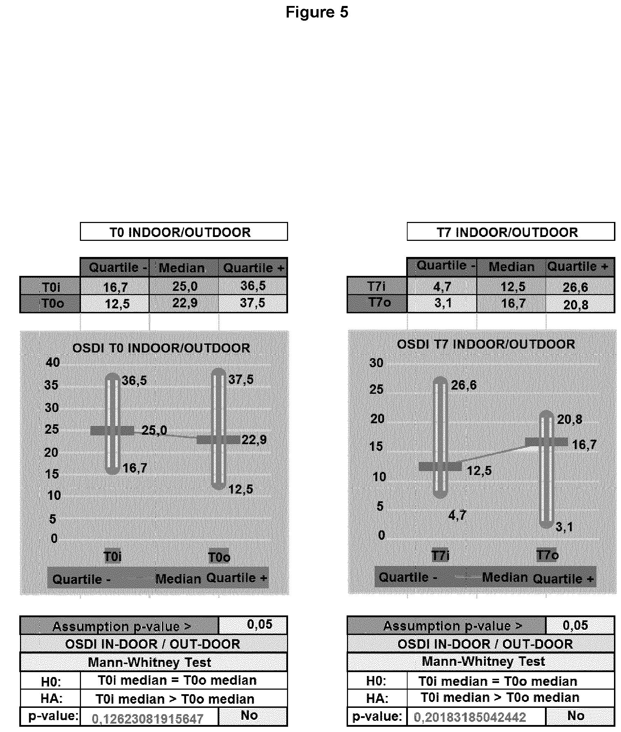

The descriptive analysis of the variables was performend through the median and the operation for verifying the objectives was performed with non-parametric tests and in particular Wilconox test for paired data and Mann-Whitney test for independent data by setting a significance level of 0.05.
The first three graphs (depicted in Fig. 4) show that the variation of OSDI index is statistically significant in all three groups under consideration (all subjects, indoor subject as well as outdoor subjects). This means that both indoor subjects and outdoor subjects showed improvement of ocular discomfort.
The forth and the fifth graph (depicted in Fig. 5) show that the improvement of ocular discomfort, assessed through OSDI questionnarie, is the same in both groups.

**Claims**

1. An ophthalmic composition in the form of ocular drops consisting of hyaluronic acid or a physiologically acceptable salt thereof, preferably sodium salt, in admixture with excipients for ophthalmic use, for use in protecting the eyes from damages due to a high level of exposure to blue light.

2. The composition for use according to claim 1 wherein the hyaluronic acid is high molecular weight hyaluronic acid, particularly with a molecular weight from 700 to 1500 KDa.

3. The composition for use according to claim 2 wherein the hyaluronic acid is hyaluronic acid with molecular weight of 900-1000 KDa.

4. The composition for use according to anyone of the previous claims wherein a hyaluronic acid salt, particularly sodium hyaluronate, is used.

5. The composition for use according to anyone of the previous claims wherein the amount (expressed as concentration) of hyaluronic acid or a physiologically acceptable salt thereof is generally from 0.1 to 0.5% w/v, more preferably from 0.2 to 0.4% w/v, still more preferably 0.4% w/v.

6. The composition for use according to anyone of the previous claims wherein the excipients are solvents such as water, isotonic agents such as sodium chloride, buffering agents such as boric acid/sodium borate, to keep the pH at a physiological value (pH about 7.2) and optionally preservatives and/or chelating agents.

7. The composition for use according to anyone of the previous claims consisting of a sterile aqueous solution at physiological pH containing 0.4% sodium hyaluronate, boric acid and sodium tetraborate, and sodium chloride.


**Patentansprüche**

1. Ophthalmische Zusammensetzung in Form von Augentropfen, bestehend aus Hyaluronsäure oder einem physiologisch annehmbaren Salz davon, vorzugsweise Natriumsalz, im Gemisch mit Exzipienten zur ophthalmischen Verwendung, zur Verwendung beim Schutz der Augen vor Schäden durch eine hohe Exposition gegenüber blauem Licht.

2. Die Zusammensetzung für den Gebrauch nach Anspruch 1, wobei die Hyaluronsäure einen hohen Hyaluronsäure mit hohem Molekulargewicht, insbesondere mit einem Molekulargewicht von 700 bis 1500 KDa.

3. Die Zusammensetzung zur Verwendung nach Anspruch 2, wobei die Hyaluronsäure Hyaluronsäure mit einem Molekulargewicht von 900-1000 KDa.

4. Die Zusammensetzungfür die Verwendung nach einem der vorhergehenden Ansprüche, wobei ein Hyaluronsäuresalz, insbesondere Natriumhyaluronat, verwendet wird.

5. Die Zusammensetzung für die Verwendung nach einem der vorhergehenden Ansprüche, wobei die Menge (ausgedrückt als Konzentration) an Hyaluronsäure oder einer physiologisch annehmbare Salz davon im Allgemeinen 0,1 bis 0,5 % (Gewicht/Volumen), bevorzugter 0,2 bis 0,4 % (Gewicht/Volumen), noch bevorzugter 0,4 % (Gewicht/-Volumen) beträgt.

6. Die Zusammensetzung für die Verwendung nach einem der vorhergehenden Ansprüche, wobei die Hilfsstoffe sind Lösungsmittel wie Wasser, isotonische Mittel wie Natriumchlorid, Puffermittel wie Borsäure/Natriumborat, um den pH-Wert auf einem physiologischen Wert zu halten. Wert (pH-Wert etwa 7,2) und gegebenenfalls Konservierungsmittel und/oder Chelatbildner.

7. Die Zusammensetzung für den Gebrauch nach einem der vorhergehenden Ansprüche, bestehend aus einem sterile wässrige Lösung mit physiologischem pH-Wert, die 0,4 % Natriumhyaluronat, Borsäure und Natriumtetraborat sowie Natriumchlorid enthält.


**Revendications**

1. . Composition ophtalmique sous forme de gouttes oculaires constituée d'acide hyaluronique ou d'un de ses sels physiologiquement acceptables, de préférence le sel de sodium, en mélange avec des excipients pour usage ophtalmique, pour la protection des yeux contre dommages dus à un niveau élevé d'exposition à la lumière bleue.

2. . La composition pour l'utilisation selon la revendication 1, dans lequel l'acide hyaluronique est à haute teneur en acide hyaluronique.l'acide hyaluronique de poids moléculaire, en particulier de 700 à 1500 KDa.

3. . La composition pour l'utilisation selon la revendication 2, dans laquelle l'acide hyaluronique est acide hyaluronique d'un poids moléculaire de 900-1000 KDa.

4. . La composition pour utilization selon l'une quelconque des revendications précédentes, dans laquelle un Le sel d'acide hyaluronique, en particulier le hyaluronate de sodium, est utilisé.

5. . La composition pour utilization selon l'une quelconque des revendications précédentes, dans laquelle le quantité (exprimée en concentration) d'acide hyaluronique ou d'un acide hyaluronique physiologique. est généralement compris entre 0,1 et 0,5 % p/v, de préférence entre 0,2 et 0,4 % p/v, de préférence encore à 0,4 % p/v.

6. .La composition pour utilization selon l'une quelconque des revendications précédentes, dans laquelle le Les excipients sont des solvants tels que l'eau, des agents isotoniques tels que le chlorure de sodium, des agents tampons tels que l'acide borique/le borate de sodium, afin de maintenir le pH à un niveau physiologique. (pH d'environ 7,2) et éventuellement des conservateurs et/ou des agents chélateurs.

7. . La composition pour l'utilisation selon l'une quelconque des revendications précédentes, consistant en une solution aqueuse stérile à pH physiologique contenant 0,4 % de hyaluronate de sodium, de l'acide borique et du tétraborate de sodium, et du chlorure de sodium.

EP 4 157 291 B1

Figure 1

Figure 2

| Patients Ref. | Q1 | | Q2 | | Q3 | | Q4 | | Q5 | | Q6 | | Q7 | | Q8 | | Q9 | | Q10 | | Q11 | | Q12 | | | |
|---|---|---|---|---|---|---|---|---|---|---|---|---|---|---|---|---|---|---|---|---|---|---|---|---|---|---|
| | T0 | T7 | T0 | T7 | T0 | T7 | T0 | T7 | T0 | T7 | T0 | T7 | T0 | T7 | T0 | T7 | T0 | T7 | T0 | T7 | T0 | T7 | T0 | T7 | T0 | TF |
| 01 | 2 | 2 | 1 | 1 | 3 | 2 | 2 | 1 | 1 | 0 | 0 | 0 | 0 | 0 | 0 | 2 | 1 | 0 | 2 | 1 | 1 | 1 | 2 | 1 | 31,3 | 22,9 |
| 02 | 1 | 0 | 0 | 0 | 1 | 0 | 0 | 0 | 0 | 0 | 0 | 0 | 0 | 0 | 0 | 0 | 0 | 0 | 0 | 0 | 0 | 0 | 0 | 0 | 4,2 | 0,0 |
| 03 | 4 | 2 | 2 | 1 | 3 | 3 | 2 | 2 | 3 | 2 | 3 | 2 | 0 | 0 | 0 | 0 | 3 | 2 | 4 | 3 | 3 | 1 | 3 | 1 | 62,5 | 39,6 |
| 04 | 1 | 2 | 0 | 1 | 1 | 2 | 0 | 0 | 0 | 0 | 1 | 0 | 1 | 0 | 0 | 0 | 1 | 0 | 2 | 2 | 3 | 2 | 1 | 1 | 22,9 | 20,8 |
| 05 | 1 | 0 | 0 | 0 | 1 | 1 | 1 | 1 | 1 | 1 | 2 | 1 | 4 | 4 | 4 | 3 | 4 | 3 | 3 | 0 | 0 | 0 | 0 | 0 | 43,8 | 29,2 |
| 06 | 0 | 1 | 0 | 0 | 2 | 2 | 1 | 2 | 2 | 2 | 1 | 3 | 1 | 2 | 2 | 2 | 1 | 2 | 1 | 3 | 2 | 2 | 3 | 2 | 33,3 | 47,9 |
| 07 | 3 | 2 | 2 | 2 | 1 | 1 | 1 | 2 | 2 | 2 | 2 | 2 | 1 | 1 | 3 | 3 | 1 | 1 | 2 | 2 | 2 | 2 | 1 | 2 | 43,8 | 45,8 |
| 08 | 1 | 0 | 1 | 0 | 1 | 1 | 1 | 0 | 0 | 1 | 2 | 2 | 2 | 1 | 3 | 1 | 2 | 1 | 2 | 1 | 1 | 0 | 1 | 0 | 35,4 | 16,7 |
| 09 | 2 | 1 | 0 | 1 | 1 | 2 | 2 | 2 | 2 | 2 | 1 | 1 | 3 | 3 | 2 | 2 | 2 | 1 | 2 | 3 | 1 | 3 | 2 | 3 | 41,7 | 50,0 |
| 10 | 1 | 0 | 0 | 1 | 1 | 0 | 2 | 1 | 2 | 0 | 1 | 1 | 1 | 1 | 2 | 1 | 2 | 1 | 2 | 1 | 2 | 1 | 2 | 1 | 37,5 | 18,8 |
| 11 | 4 | 1 | 4 | 1 | 3 | 2 | 4 | 2 | 3 | 1 | 3 | 1 | 3 | 2 | 4 | 1 | 4 | 2 | 3 | 3 | 4 | 1 | 4 | 1 | 89,6 | 37,5 |
| 12 | 0 | 0 | 0 | 0 | 0 | 0 | 0 | 0 | 0 | 0 | 1 | 1 | 0 | 1 | 1 | 1 | 1 | 1 | 1 | 1 | 1 | 1 | 1 | 1 | 12,5 | 14,6 |
| 13 | 2 | 1 | 2 | 1 | 2 | 2 | 2 | 0 | 2 | 1 | 2 | 1 | 2 | 2 | 2 | 1 | 2 | 0 | 2 | 0 | 2 | 1 | 2 | 0 | 50,0 | 20,8 |
| 14 | 3 | 2 | 2 | 1 | 1 | 1 | 3 | 1 | 1 | 1 | 4 | 3 | 3 | 2 | 3 | 2 | 3 | 2 | 3 | 2 | 3 | 2 | 3 | 2 | 66,7 | 43,8 |
| 15 | 2 | 1 | 2 | 1 | 1 | 0 | 0 | 0 | 1 | 0 | 2 | 1 | 2 | 1 | 1 | 1 | 1 | 1 | 2 | 1 | 3 | 1 | 3 | 2 | 41,7 | 20,8 |
| 16 | 1 | 0 | 2 | 0 | 1 | 0 | 1 | 1 | 1 | 0 | 1 | 1 | 0 | 0 | 1 | 1 | 1 | 0 | 1 | 1 | 0 | 0 | 0 | 0 | 20,8 | 8,3 |
| 17 | 0 | 0 | 1 | 0 | 1 | 0 | 1 | 0 | 1 | 0 | 1 | 0 | 0 | 0 | 0 | 0 | 0 | 0 | 0 | 0 | 0 | 0 | 0 | 0 | 10,4 | 0,0 |
| 18 | 2 | 2 | 0 | 1 | 1 | 1 | 1 | 0 | 0 | 0 | 0 | 0 | 0 | 0 | 1 | 0 | 0 | 0 | 1 | 1 | 0 | 1 | 0 | 0 | 12,5 | 12,5 |
| 19 | 0 | 0 | 0 | 0 | 0 | 0 | 1 | 1 | 1 | 1 | 1 | 1 | 0 | 0 | 0 | 0 | 1 | 0 | 0 | 1 | 1 | 1 | 0 | 0 | 10,4 | 10,4 |
| 20 | 1 | 0 | 2 | 1 | 2 | 1 | 2 | 0 | 1 | 0 | 2 | 0 | 2 | 1 | 2 | 1 | 2 | 1 | 2 | 1 | 2 | 1 | 1 | 0 | 43,8 | 14,6 |
| 21 | 1 | 0 | 0 | 0 | 1 | 0 | 0 | 0 | 0 | 0 | 0 | 0 | 1 | 0 | 1 | 0 | 1 | 0 | 0 | 0 | 0 | 0 | 0 | 0 | 10,4 | 0,0 |
| 22 | 2 | 1 | 2 | 0 | 2 | 0 | 0 | 0 | 2 | 1 | 1 | 1 | 0 | 0 | 1 | 1 | 1 | 1 | 3 | 1 | 2 | 2 | 1 | 0 | 35,4 | 16,7 |
| 23 | 1 | 0 | 1 | 0 | 0 | 0 | 0 | 0 | 0 | 0 | 1 | 0 | 1 | 0 | 1 | 0 | 2 | 0 | 1 | 0 | 2 | 0 | 1 | 0 | 22,9 | 0,0 |
| 24 | 1 | 1 | 0 | 1 | 0 | 1 | 0 | 0 | 0 | 1 | 1 | 1 | 1 | 1 | 1 | 1 | 1 | 1 | 0 | 1 | 1 | 1 | 0 | 2 | 12,5 | 25,0 |
| 25 | 1 | 1 | 0 | 1 | 0 | 1 | 0 | 0 | 1 | 0 | 1 | 0 | 0 | 2 | 1 | 2 | 0 | 2 | 0 | 1 | 1 | 0 | 0 | 0 | 10,4 | 20,8 |
| 26 | 1 | 0 | 2 | 1 | 1 | 0 | 2 | 1 | 2 | 1 | 2 | 1 | 1 | 0 | 2 | 1 | 2 | 1 | 2 | 1 | 1 | 0 | 2 | 1 | 41,7 | 16,7 |
| 27 | 3 | 0 | 0 | 0 | 0 | 0 | 0 | 0 | 0 | 0 | 0 | 0 | 1 | 0 | 1 | 0 | 0 | 0 | 0 | 0 | 0 | 0 | 0 | 0 | 10,4 | 0,0 |
| 28 | 1 | 0 | 1 | 0 | 0 | 0 | 1 | 0 | 0 | 0 | 0 | 0 | 0 | 0 | 0 | 0 | 0 | 0 | 1 | 1 | 0 | 1 | 1 | 1 | 10,4 | 6,3 |
| 29 | 1 | 1 | 0 | 1 | 1 | 1 | 1 | 1 | 0 | 2 | 1 | 1 | 1 | 0 | 2 | 2 | 1 | 0 | 0 | 0 | 0 | 0 | 0 | 0 | 16,7 | 18,8 |
| 30 | 1 | 1 | 0 | 0 | 1 | 1 | 0 | 0 | 0 | 0 | 1 | 0 | 1 | 0 | 2 | 1 | 0 | 0 | 1 | 1 | 1 | 0 | 0 | 0 | 16,7 | 8,3 |
| 31 | 0 | 0 | 0 | 0 | 2 | 1 | 1 | 1 | 1 | 0 | 1 | 0 | 0 | 0 | 1 | 0 | 1 | 1 | 0 | 0 | 1 | 1 | 1 | 1 | 18,8 | 10,4 |
| 32 | 1 | 0 | 1 | 0 | 1 | 0 | 1 | 0 | 1 | 0 | 1 | 0 | 1 | 0 | 1 | 0 | 1 | 0 | 1 | 0 | 1 | 0 | 1 | 0 | 25,0 | 0,0 |
| 33 | 3 | 1 | 3 | 0 | 2 | 0 | 2 | 0 | 1 | 0 | 0 | 0 | 0 | 0 | 1 | 0 | 1 | 0 | 2 | 0 | 2 | 0 | 1 | 0 | 37,5 | 2,1 |
| 34 | 2 | 2 | 2 | 2 | 1 | 1 | 3 | 2 | 2 | 2 | 2 | 3 | 4 | 3 | 1 | 1 | 2 | 2 | 2 | 3 | 3 | 1 | 2 | 1 | 54,2 | 47,9 |
| 35 | 1 | 1 | 1 | 1 | 1 | 1 | 2 | 1 | 1 | 1 | 2 | 1 | 2 | 1 | 2 | 1 | 2 | 1 | 1 | 1 | 2 | 1 | 2 | 1 | 39,6 | 25,0 |
| 36 | 2 | 0 | 2 | 0 | 2 | 0 | 0 | 0 | 0 | 0 | 3 | 0 | 0 | 0 | 2 | 0 | 0 | 0 | 1 | 0 | 1 | 0 | 4 | 0 | 35,4 | 0,0 |
| 37 | 0 | 0 | 0 | 0 | 0 | 0 | 0 | 0 | 1 | 0 | 1 | 0 | 1 | 0 | 1 | 1 | 0 | 0 | 0 | 0 | 1 | 0 | 0 | 0 | 10,4 | 2,1 |
| 38 | 1 | 1 | 1 | 1 | 2 | 1 | 1 | 0 | 0 | 0 | 0 | 0 | 0 | 0 | 0 | 0 | 0 | 0 | 1 | 0 | 1 | 0 | 1 | 0 | 16,7 | 6,3 |
| 39 | 1 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 2,1 | 2,1 |
| 40 | 0 | 1 | 1 | 1 | 0 | 1 | 1 | 0 | 1 | 1 | 1 | 0 | 5 | 1 | 1 | 1 | 1 | 1 | 1 | 0 | 1 | 1 | 1 | 0 | 29,2 | 16,7 |
| 41 | 2 | 1 | 2 | 1 | 1 | 0 | 1 | 0 | 0 | 0 | 1 | 2 | 1 | 2 | 2 | 1 | 0 | 0 | 0 | 0 | 4 | 1 | 2 | 1 | 33,3 | 18,8 |
| 42 | 1 | 0 | 0 | 0 | 1 | 0 | 0 | 0 | 0 | 0 | 0 | 0 | 1 | 0 | 1 | 0 | 1 | 0 | 0 | 0 | 0 | 0 | 0 | 0 | 10,4 | 0,0 |
| 43 | 2 | 1 | 2 | 0 | 2 | 0 | 0 | 0 | 2 | 1 | 1 | 1 | 0 | 0 | 1 | 1 | 1 | 1 | 3 | 1 | 2 | 2 | 1 | 0 | 35,4 | 16,7 |
| 44 | 0 | 0 | 0 | 0 | 0 | 0 | 0 | 0 | 0 | 0 | 1 | 0 | 1 | 0 | 1 | 0 | 2 | 0 | 1 | 0 | 2 | 0 | 1 | 0 | 18,8 | 0,0 |
| 45 | 1 | 1 | 0 | 1 | 0 | 1 | 0 | 0 | 0 | 1 | 1 | 1 | 1 | 1 | 1 | 1 | 1 | 1 | 0 | 1 | 1 | 1 | 0 | 2 | 12,5 | 25,0 |
| 46 | 2 | 1 | 0 | 1 | 0 | 1 | 0 | 0 | 1 | 0 | 1 | 0 | 0 | 2 | 1 | 2 | 0 | 2 | 0 | 1 | 1 | 0 | 0 | 0 | 12,5 | 20,8 |
| 47 | 1 | 1 | 1 | 1 | 1 | 0 | 1 | 1 | 2 | 1 | 2 | 1 | 1 | 0 | 2 | 1 | 2 | 1 | 2 | 1 | 1 | 0 | 2 | 1 | 37,5 | 18,8 |
| 48 | 3 | 0 | 0 | 0 | 0 | 0 | 0 | 0 | 0 | 0 | 0 | 0 | 1 | 0 | 1 | 0 | 0 | 0 | 0 | 0 | 0 | 0 | 0 | 0 | 10,4 | 0,0 |
| 49 | 1 | 0 | 1 | 0 | 0 | 0 | 1 | 0 | 0 | 0 | 0 | 0 | 0 | 0 | 0 | 0 | 0 | 0 | 1 | 1 | 0 | 1 | 1 | 1 | 10,4 | 6,3 |
| 50 | 2 | 1 | 2 | 1 | 0 | 1 | 1 | 1 | 0 | 2 | 1 | 1 | 1 | 0 | 2 | 2 | 1 | 0 | 0 | 0 | 0 | 0 | 0 | 0 | 20,8 | 18,8 |

Figure 3

| Patients Ref. | Q1 | | Q2 | | Q3 | | Q4 | | Q5 | | Q6 | | Q7 | | Q8 | | Q9 | | Q10 | | Q11 | | Q12 | | T0 | TF |
|---|---|---|---|---|---|---|---|---|---|---|---|---|---|---|---|---|---|---|---|---|---|---|---|---|---|---|
| | T0 | T7 | T0 | T7 | T0 | T7 | T0 | T7 | T0 | T7 | T0 | T7 | T0 | T7 | T0 | T7 | T0 | T7 | T0 | T7 | T0 | T7 | T0 | T7 | | |
| 51 | 1 | 1 | 0 | 0 | 1 | 1 | 0 | 0 | 0 | 0 | 1 | 0 | 1 | 0 | 2 | 1 | 0 | 0 | 1 | 1 | 1 | 0 | 0 | 0 | 16.7 | 8.3 |
| 52 | 2 | 0 | 1 | 0 | 0 | 1 | 0 | 0 | 0 | 0 | 0 | 0 | 0 | 0 | 1 | 1 | 0 | 1 | 0 | 0 | 0 | 0 | 0 | 1 | 8.3 | 8.3 |
| 53 | 1 | 1 | 1 | 0 | 1 | 0 | 0 | 0 | 0 | 0 | 1 | 0 | 1 | 0 | 1 | 0 | 1 | 0 | 1 | 0 | 1 | 0 | 1 | 0 | 20.8 | 2.1 |
| 54 | 1 | 0 | 1 | 0 | 1 | 0 | 1 | 0 | 1 | 0 | 1 | 0 | 1 | 0 | 1 | 0 | 1 | 0 | 1 | 0 | 1 | 0 | 1 | 0 | 25.0 | 0.0 |
| 55 | 3 | 2 | 3 | 1 | 2 | 2 | 2 | 1 | 2 | 2 | 2 | 2 | 2 | 2 | 1 | 1 | 2 | 2 | 2 | 2 | 3 | 2 | 3 | 2 | 56.3 | 43.8 |
| 56 | 1 | 0 | 1 | 0 | 0 | 0 | 0 | 0 | 0 | 0 | 0 | 0 | 0 | 0 | 1 | 1 | 0 | 0 | 1 | 1 | 0 | 0 | 0 | 0 | 8.3 | 4.2 |
| 57 | 2 | 1 | 1 | 0 | 1 | 1 | 0 | 0 | 0 | 0 | 0 | 1 | 0 | 0 | 1 | 1 | 0 | 0 | 1 | 1 | 1 | 1 | 1 | 1 | 16.7 | 14.6 |
| 58 | 2 | 1 | 0 | 0 | 2 | 1 | 1 | 1 | 0 | 0 | 0 | 0 | 0 | 0 | 2 | 2 | 0 | 0 | 1 | 1 | 1 | 1 | 0 | 0 | 18.8 | 14.6 |
| 59 | 3 | 3 | 2 | 2 | 3 | 2 | 2 | 2 | 2 | 2 | 2 | 1 | 3 | 3 | 3 | 1 | 2 | 2 | 3 | 1 | 2 | 2 | 3 | 1 | 62.5 | 45.8 |
| 60 | 1 | 0 | 1 | 1 | 2 | 1 | 1 | 1 | 1 | 1 | 1 | 1 | 1 | 1 | 1 | 1 | 1 | 1 | 1 | 1 | 1 | 1 | 1 | 1 | 27.1 | 22.9 |
| 61 | 1 | 0 | 1 | 1 | 1 | 0 | 1 | 1 | 1 | 1 | 1 | 1 | 1 | 1 | 1 | 0 | 1 | 1 | 1 | 1 | 1 | 1 | 1 | 1 | 25.0 | 18.8 |
| 62 | 0 | 0 | 0 | 0 | 0 | 0 | 1 | 0 | 0 | 0 | 0 | 0 | 0 | 0 | 0 | 0 | 0 | 0 | 0 | 0 | 0 | 0 | 0 | 0 | 2.1 | 0.0 |
| 63 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 25.0 | 25.0 |
| 64 | 1 | 0 | 1 | 0 | 1 | 0 | 1 | 0 | 1 | 0 | 1 | 0 | 1 | 0 | 1 | 0 | 1 | 0 | 1 | 0 | 1 | 0 | 1 | 0 | 25.0 | 0.0 |
| 65 | 3 | 1 | 3 | 2 | 3 | 0 | 3 | 1 | 3 | 1 | 2 | 1 | 2 | 1 | 2 | 1 | 2 | 1 | 2 | 1 | 2 | 1 | 2 | 1 | 60.4 | 25.0 |
| 66 | 1 | 1 | 1 | 2 | 1 | 1 | 2 | 1 | 1 | 1 | 1 | 1 | 1 | 1 | 1 | 2 | 2 | 2 | 1 | 1 | 1 | 1 | 1 | 1 | 29.2 | 31.3 |
| 67 | 0 | 0 | 1 | 0 | 1 | 0 | 1 | 0 | 1 | 0 | 1 | 0 | 1 | 0 | 1 | 0 | 1 | 0 | 1 | 0 | 1 | 0 | 1 | 0 | 22.9 | 0.0 |
| 68 | 2 | 1 | 1 | 1 | 1 | 1 | 1 | 1 | 1 | 1 | 1 | 1 | 1 | 1 | 1 | 1 | 1 | 1 | 2 | 1 | 1 | 1 | 1 | 1 | 29.2 | 25.0 |
| 69 | 2 | 1 | 0 | 1 | 2 | 1 | 1 | 1 | 1 | 1 | 1 | 2 | 1 | 1 | 1 | 2 | 2 | 2 | 1 | 1 | 2 | 2 | 2 | 2 | 33.3 | 35.4 |
| 70 | 2 | 0 | 1 | 0 | 0 | 0 | 0 | 0 | 0 | 0 | 0 | 0 | 0 | 0 | 0 | 0 | 0 | 0 | 0 | 0 | 0 | 0 | 0 | 0 | 6.3 | 0.0 |
| 71 | 2 | 1 | 2 | 0 | 2 | 1 | 1 | 1 | 2 | 2 | 2 | 2 | 2 | 2 | 1 | 2 | 3 | 1 | 2 | 1 | 2 | 1 | 0 | 0 | 43.8 | 29.2 |
| 72 | 1 | 1 | 0 | 0 | 0 | 0 | 0 | 0 | 0 | 0 | 0 | 0 | 0 | 0 | 1 | 0 | 1 | 0 | 0 | 1 | 0 | 0 | 1 | 0 | 8.3 | 4.2 |
| 73 | 3 | 3 | 2 | 3 | 2 | 2 | 2 | 3 | 2 | 2 | 2 | 3 | 2 | 2 | 2 | 2 | 2 | 2 | 2 | 2 | 1 | 1 | 2 | 2 | 50.0 | 56.3 |
| 74 | 1 | 0 | 0 | 0 | 1 | 0 | 0 | 0 | 0 | 0 | 0 | 0 | 0 | 0 | 1 | 1 | 0 | 0 | 1 | 1 | 2 | 1 | 1 | 1 | 14.6 | 8.3 |
| 75 | 2 | 2 | 1 | 1 | 2 | 1 | 1 | 2 | 2 | 2 | 2 | 1 | 2 | 2 | 3 | 2 | 2 | 1 | 1 | 1 | 2 | 2 | 2 | 1 | 45.8 | 37.5 |
| 76 | 3 | 2 | 2 | 2 | 3 | 2 | 3 | 3 | 3 | 3 | 2 | 3 | 4 | 3 | 3 | 3 | 2 | 2 | 3 | 3 | 3 | 3 | 2 | 2 | 68.8 | 64.6 |
| 77 | 0 | 0 | 0 | 0 | 0 | 0 | 0 | 0 | 0 | 0 | 0 | 0 | 0 | 0 | 1 | 1 | 1 | 1 | 0 | 0 | 0 | 0 | 0 | 0 | 4.2 | 4.2 |
| 78 | 3 | 1 | 2 | 1 | 3 | 3 | 1 | 1 | 1 | 1 | 1 | 3 | 3 | 3 | 3 | 3 | 1 | 1 | 3 | 3 | 4 | 3 | 0 | 0 | 52.1 | 47.9 |
| 79 | 0 | 0 | 0 | 0 | 0 | 0 | 0 | 0 | 0 | 0 | 0 | 1 | 0 | 0 | 0 | 1 | 0 | 0 | 1 | 1 | 1 | 0 | 0 | 0 | 4.2 | 6.3 |
| 80 | 1 | 0 | 0 | 0 | 1 | 0 | 1 | 0 | 1 | 0 | 1 | 0 | 0 | 0 | 2 | 0 | 1 | 0 | 1 | 0 | 1 | 0 | 0 | 0 | 20.8 | 0.0 |
| 81 | 0 | 0 | 1 | 0 | 0 | 1 | 2 | 2 | 0 | 0 | 0 | 0 | 2 | 0 | 0 | 0 | 0 | 0 | 0 | 0 | 0 | 2 | 0 | 0 | 10.4 | 10.4 |
| 82 | 3 | 1 | 0 | 0 | 3 | 1 | 0 | 0 | 1 | 0 | 1 | 0 | 0 | 0 | 2 | 1 | 1 | 0 | 1 | 1 | 0 | 0 | 3 | 1 | 31.3 | 10.4 |
| 83 | 1 | 0 | 2 | 0 | 3 | 1 | 1 | 1 | 1 | 1 | 0 | 0 | 0 | 1 | 1 | 0 | 1 | 1 | 1 | 0 | 1 | 1 | 1 | 0 | 27.1 | 12.5 |
| 84 | 0 | 0 | 2 | 0 | 0 | 0 | 0 | 0 | 0 | 0 | 0 | 0 | 0 | 0 | 0 | 0 | 1 | 0 | 1 | 1 | 1 | 1 | 0 | 0 | 10.4 | 4.2 |
| 85 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 25.0 | 25.0 |
| 86 | 1 | 0 | 3 | 1 | 0 | 0 | 1 | 1 | 0 | 0 | 1 | 1 | 0 | 0 | 1 | 1 | 0 | 0 | 1 | 0 | 1 | 0 | 1 | 0 | 20.8 | 8.3 |
| 87 | 3 | 1 | 3 | 2 | 2 | 1 | 3 | 2 | 2 | 2 | 1 | 1 | 1 | 1 | 1 | 1 | 1 | 1 | 1 | 1 | 1 | 1 | 0 | 0 | 39.6 | 29.2 |
| 88 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0.0 | 0.0 |
| 89 | 0 | 0 | 0 | 0 | 3 | 1 | 1 | 1 | 1 | 0 | 2 | 0 | 0 | 0 | 4 | 1 | 1 | 0 | 3 | 0 | 2 | 0 | 1 | 0 | 37.5 | 6.3 |
| 90 | 2 | 1 | 1 | 0 | 1 | 2 | 0 | 1 | 1 | 0 | 1 | 0 | 0 | 0 | 2 | 0 | 1 | 0 | 1 | 0 | 1 | 0 | 1 | 0 | 25.0 | 8.3 |
| 91 | 3 | 2 | 1 | 0 | 2 | 1 | 1 | 0 | 3 | 0 | 3 | 2 | 4 | 2 | 3 | 1 | 3 | 1 | 1 | 5 | 1 | 1 | 5 | 0 | 62.5 | 31.3 |
| 92 | 1 | 0 | 0 | 0 | 1 | 0 | 1 | 0 | 1 | 0 | 1 | 0 | 0 | 0 | 1 | 0 | 0 | 0 | 1 | 0 | 1 | 0 | 0 | 0 | 16.7 | 0.0 |
| 93 | 0 | 0 | 0 | 1 | 1 | 1 | 0 | 0 | 0 | 0 | 0 | 0 | 0 | 0 | 0 | 0 | 0 | 0 | 1 | 1 | 0 | 0 | 0 | 0 | 4.2 | 6.3 |
| 94 | 2 | 1 | 0 | 0 | 1 | 1 | 1 | 1 | 1 | 1 | 1 | 1 | 1 | 0 | 1 | 1 | 0 | 0 | 0 | 0 | 3 | 2 | 2 | 1 | 27.1 | 18.8 |
| 95 | 1 | 1 | 0 | 0 | 1 | 0 | 2 | 1 | 2 | 1 | 1 | 1 | 2 | 0 | 3 | 1 | 1 | 0 | 2 | 0 | 3 | 1 | 2 | 0 | 41.7 | 12.5 |
| 96 | 1 | 1 | 0 | 0 | 1 | 0 | 1 | 0 | 0 | 1 | 1 | 1 | 0 | 0 | 1 | 1 | 1 | 1 | 1 | 0 | 0 | 1 | 1 | 0 | 16.7 | 12.5 |
| 97 | 1 | 1 | 0 | 0 | 1 | 1 | 0 | 0 | 1 | 0 | 0 | 1 | 1 | 0 | 1 | 1 | 1 | 0 | 2 | 0 | 1 | 0 | 0 | 0 | 18.8 | 8.3 |
| 98 | 2 | 2 | 2 | 2 | 3 | 2 | 2 | 2 | 2 | 2 | 3 | 2 | 3 | 2 | 3 | 2 | 2 | 3 | 3 | 2 | 3 | 2 | 3 | 2 | 64.6 | 52.1 |
| 99 | 2 | 1 | 4 | 3 | 1 | 1 | 1 | 1 | 0 | 0 | 0 | 0 | 0 | 0 | 0 | 0 | 0 | 2 | 0 | 2 | 3 | 0 | 2 | 3 | 27.1 | 27.1 |
| 100 | 1 | 1 | 1 | 1 | 1 | 1 | 0 | 0 | 1 | 1 | 1 | 1 | 1 | 1 | 2 | 1 | 2 | 1 | 0 | 0 | 1 | 0 | 0 | 0 | 22.9 | 16.7 |

## Figure 4

### OSDI all patients

| | Quartile - | Median | Quartile + |
|---|---|---|---|
| T0 | 12,5 | 25,0 | 37,5 |
| T7 | 4,2 | 14,6 | 25,0 |

| Assumption p-value > | 0,05 |
|---|---|
| OSDI all patients | |
| Wilcoxon Signed Rank Test | |

| H0: | T0 median = T7 median | |
|---|---|---|
| HA: | T0 median > T7 median | |
| p-value: | 0,00000000000276 | Yes |

**ODSI all patients**

40
35 — 37,5
30
25 — 25,0 — 25,0
20
15 — 14,6
10 — 12,5
5 — 4,2
0

T0　　　T7

Quartile - ——— Median Quartile +

| OSDI "Indoor" | |
|---|---|
| Wilcoxon Signed Rank Test | |

| H0: | T0 median = T7 median | |
|---|---|---|
| HA: | T0 median > T7 median | |
| p-value: | 0,00000003929524 | Yes |

| OSDI "Outdoor" | |
|---|---|
| Wilcoxon Signed Rank Test | |

| H0: | T0 median = T7 median | |
|---|---|---|
| HA: | T0 median > T7 median | |
| p-value: | 0,00000227297325 | Yes |

### OSDI "Indoor"

| | Quartile - | Median | Quartile + |
|---|---|---|---|
| T0 | 16,7 | 25,0 | 36,5 |
| T7 | 4,7 | 12,5 | 26,6 |

**OSDI "Indoor"**

40
35 — 36,5
30
25 — 25,0 — 26,6
20
15 — 16,7 — 12,5
10
5 — 4,7
0

T0　　　T7

Quartile - ——— Median Quartile +

### OSDI "Outdoor"

| | Quartile - | Median | Quartile + |
|---|---|---|---|
| T0 | 12,5 | 22,9 | 37,5 |
| T7 | 3,1 | 16,7 | 20,8 |

**OSDI "Outdoor"**

40
35 — 37,5
30
25
20 — 22,9 — 20,8
15 — 16,7
10 — 12,5
5
0 — 3,1

T0　　　T7

Quartile - ——— Median Quartile +

EP 4 157 291 B1

**Figure 5**

**T0 INDOOR/OUTDOOR**

|  | Quartile - | Median | Quartile + |
|---|---|---|---|
| T0i | 16,7 | 25,0 | 36,5 |
| T0o | 12,5 | 22,9 | 37,5 |

**OSDI T0 INDOOR/OUTDOOR**

| Assumption p-value > | | 0,05 |
|---|---|---|
| OSDI IN-DOOR / OUT-DOOR | | |
| Mann-Whitney Test | | |
| H0: | T0i median = T0o median | |
| HA: | T0i median > T0o median | |
| p-value: | 0,12623081915647 | No |

**T7 INDOOR/OUTDOOR**

|  | Quartile - | Median | Quartile + |
|---|---|---|---|
| T7i | 4,7 | 12,5 | 26,6 |
| T7o | 3,1 | 16,7 | 20,8 |

**OSDI T7 INDOOR/OUTDOOR**

| Assumption p-value > | | 0,05 |
|---|---|---|
| OSDI IN-DOOR / OUT-DOOR | | |
| Mann-Whitney Test | | |
| H0: | T0i median = T0o median | |
| HA: | T0i median > T0o median | |
| p-value: | 0,20183185042442 | No |

13

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2016181342 A
- IT 102015000065559
- WO 2016162809 A

**Non-patent literature cited in the description**

- **PAULOIN et al.** *Molecular Vision*, 2009, vol. 15, 577-583
- **LI et al.** *Environmental and Molecular Mutagenesis*, 2013, vol. 54, 429-449
- **KOJIMA et al.** *International Journal of Molecular Sciences*, 2020, vol. 21, 3516